# EUROPEAN PATENT APPLICATION

(11) **EP 0 756 871 A1**
(43) Date of publication of application: **05.02.1997**
(21) Application number: 95870092.4
(22) Date of filing: 01.08.1995
(51) Int. Cl.: A61K 38/20, A61K 39/395, A61K 45/00

(54) **Use of a pharmaceutical composition comprising an effective amount of interleukin-10, an analog and/or an agonist of interleukin-10**

(71) Applicant: INSTITUT PASTEUR, F-75724 Paris Cédex 15 (FR); INSTITUT NATIONAL DE LA SANTE ET DE LA RECHERCHE MEDICALE (INSERM), 75654 Paris Cédex 13 (FR); UNIVERSITE LIBRE DE BRUXELLES, B-1050 Bruxelles (BE)
(72) Inventor: Pretolani, Marina, F-75015 Paris (FR); Zuany-Amorin, Claudia, F-93100 Montreuil (FR); Lefort, Jean, F-92110 Clichy (FR); Vargaftig, Boris, F-75015 Paris (FR)
(74) Representative: Van Malderen, Joelle

(57) **Abstract**

Use of an element chosen among the group consisting of interleukin-10, an analog and/or an agonist thereof, for the manufacturing of a medicament, for the treatment and/or the prevention of diseases involving the in vivo modulation of the biological activity of interleukin-5.

## Description

### Field of the invention.

The present invention concerns the use of a pharmaceutical composition comprising an effective amount of an element chosen among the group consisting of interleukin-10 (IL-10), an analog and/or an agonist of interleukin-10, for the manufacturing of a medicament intended for the treatment or the prevention of diseases involving the in vivo modulation of the biological activity of interleukin-5, especially diseases induced by an excessive amount of interleukin-5 release by T-lymphocytes.

The present invention concerns also a pharmaceutical composition comprising an effective amount of an element chosen among the group consisting of interleukin-10, an analog and/or an agonist thereof, an effective amount of a broncho-dilator and/or an effective amount of an anti-histamine compound for the treatment or the prevention of allergic diseases.

### Background of the invention and state of the art.

Human IL-10 is a cytokine which, in its mature form, has 160 amino acids, and contains 2 intramolecular disulphide bridges. It also contains a glycosylation site, but is not glycosylated.

IL-10 is produced in the body by a number of different cell type. Cells involved in the immune system, namely lymphocytes T and B and monocytes / macrophaqes, represent important sources of IL-10. Outside the immune system, keratinocytes and certain tumour cells are also capable of producing IL-10. The factors controlling IL-10 synthesis are not completely understood.

IL-10 is a cytokine originally known as Cytokine Synthesis Inhibitor Factor (CSIF) which exerces its biological effects on a number of different target cells.

The human IL-10 has been already described in the patent application WO91/00349.

The international patent application WO94/17773 describes the use of interleukin-10 (IL-10) or analogs and agonists thereof for the treatment of the prevention of diseases involving interleukin-5 (IL-5) release by T-lymphocytes, especially diseases involving IL-5 overrelease by T-lymphocytes, such as diseases associated with eosinophilia, selected from the group consisting of : atopic diseases including allergic rhinitis and bronchial asthma, atopic dermatitis and other cutaneous diseases associated with eosinophil infiltration (including chronic eczema, erythema multiforme, dermatitis herpetiformis, mastocytosis, bullous disorders), Crohn's disease, broncho-pulmonary aspergillosis, Spanish toxic oil syndrome, myalgiaeosinophilia syndrome induced by L-tryptophan preparations, certain drugs allergic reactions, tropical eosinophilia, helminthic diseases, Schulman syndrome (eosinophilic fasciitis), Churg-Strauss syndrome and other vasculitides with eosinophilia, Loeffler syndrome, chronic eosinophilic pneumonia, eosinophilic gastroenteritis, and the hypereosinophilic syndrome.

In said medicament, IL-10 is either the sole active principle or is associated with cyclosporin A or other related immunosuppressive agents (such as FK 506, rapamycin or anti-CD3 monoclonal antibody), or comprises a usual pharmaceutical carrier (a non-toxic substance suitable for delivering a pharmaceutical composition to a patient).

Said patent application demonstrates that B7/CD28-dependent IL-5 release by human resting T-cells is inhibited by IL-10 in vitro. IL-10 was found to inhibit IL-5 release in a dose-dependent manner in T-cells activated with either anti-CD3 monoclonal antibodies cross-linked on B7/CD32-transfected mouse fibroblasts or by p.m.A. in conjunction with anti-CD28 monoclonal antibodies. More specifically, said patent application shows that in vitro a recombinant IL-10 inhibits IL-5 release by human resting T-cells costimulated by B7/CD28 signalling; that a recombinant IL-10 reduces IL-5 mRNA accumulation in T-cells activated by PMA and anti-CD28 monoclonal antibodies; that a recombinant IL-10 does not inhibit IL-5 release in response to PMA and A23187 calcium ionophore and that endogenous IL-10 down-regulates IL-5 release by resting T-cells.

However, these results are restricted to in vitro assays and the mechanism that regulates in vivo antigen-induced eosinophil recruitment and modulation of the biological activity of IL-5 is still unknown.

It is also known that in vitro, IL-10 specifically suppresses IFN-γ production by TH1 cells (A. Chernoff et al., Journal of Immunology, pp. 5493-5499 (1994)) and that IFN-γ regulates in vitro antigen-induced eosinophil recruitment (by inhibiting the infiltration and the proliferation of CD4⁺ T-cells, producing IL-4, IL-5 and IL-6) (I. Iwamoto et al., Journal of Exp. Med., 177, pp. 573-576 (1993) et Boguniewicz et al., Clinical and Experimental Allergy, vol. 23, pp. 785-790 (1993)).

Consequently it is extremely difficult for a man skilled in the art to predict the effect of IL-10 in vivo upon diseases involving interleukin-5 release by T-lymphocytes.

In addition, specific allergic diseases, such as asthma are treated by administration of broncho-dilators such as β₂-agonists (isoprenaline, feneterol, salbutamol, salmeterol, formoterol, ...) or phospho-diesterase inhibitors (Theophylline) (P. J. Barnes, Clinical and Experimental Allergy, vol. 23, pp. 165-167 (1993)).

However, the administration of broncho-dilators may present the following problems : the development of tolerance due to β-receptors desensitization and the pharmacological consequences of β-receptors stimulation (including increased heart rate, tremor, dysrhythmia and hypoxemia). High doses of β-agonists may be also associated with increased mortality (GLAXO Review, European Respiratory Society Annual Congress, Vienna (September 1992)).

In addition, it is often necessary to treat also the asthma with anti-inflammatory drugs such as corticosteroids, or other specific allergic diseases with anti-histamine compounds. Recent National Institutes of Health (NIH) guidelines for the treatment of asthma have focused on early intervention with anti-inflammatory therapy, particularly inhaled glucocorticoids (International Consensus Report on Diagnostic and Management of Asthma, June 92, US Department of Health and Human Services, Public Health Service, National Institutes of Health , Publication No. 92-3091). However, a subset of asthmatics fails to demonstrate a satisfactory response even to systemic glucocorticoids therapy (Carmichael et al., Br. Med. J. 282, pp. 1419-1422 (1981)) and has been termed "steroid resistant (SR)".

It is important to recognize these patients early because failure to respond to steroids often leads to courses of very high doses glucocorticoid therapy and consequent adverse effects despite persistent airway compromise (Kamada et al., Pediatr. Pulmonol. 14, pp. 180-186 (1992)).

There is thus also a need for new or improved treatments of specific allergic diseases which do not prevent or which may reduce the drawbacks of the drugs already used either as broncho-dilators or as anti-inflammatory drugs or as anti-histamine compounds.

### Aims of the invention.

The main aim of the invention is to obtain a new method and pharmaceutical composition for the treatment or the prevention of diseases involving the in vivo modulation of the biological activity of interleukin-5, e.g. interleukin-5 release by T-lymphocytes.

A further aim of the invention is to provide a pharmaceutical composition which may improve the treatments of specific allergic disease such as asthma, which do not prevent or which may reduce the drawbacks of drugs already used as broncho-dilators, anti-inflammatory drugs and/or anti-histamine compounds.

### Summary of the invention.

The solution of the above technical problem has been discovered by the present invention to reside into the administration to patients of an element chosen among the group consisting of interleukin-10 (IL-10), an analog and/or a agonist thereof, either alone or in combination with other treatments.

The present invention concerns specifically the use of a pharmaceutical composition comprising an element chosen among the group consisting of interleukin-10 (IL-10), an analog and/or a agonist thereof, for the manufacturing of a medicament, for the treatment and/or the prevention of a disease involving the in vivo modulation of the biological activity of interleukin-5.

By the term "interleukin-10 (IL-10)" is meant a pure mammalian, particularly human, interleukin-10 as purified from natural sources, or preferably as produced by expression in a suitable host of a recombinant DNA sequence encoding interleukin-10. The IL-10 of the invention also includes the viral form of interleukin-10, chimeric proteins composed of sequences of human and viral IL-10 origin, provided that the properties of IL-10 as required by the present invention are maintained. "IL-10" also englobes analogs and peptides of interleukin-10 carrying the IL-10 activity, as described for example in the PCT publication WO91/00349 as a cytokine synthesis inhibitory factor.

As suitable recombinant hosts, mention is made of eukaryotic cells transfected by the suitable vectors capable of expressing IL-10 activity in vivo, and particularly retroviral vectors carrying the appropriate IL-10 encoding sequence(s) or a baculoviral system. Retroviral vectors are introduced in mammalian cells (for example CHO cells). The recombinant protein obtained in the supernatant is similar to the native protein expressed in mammals. Moreover, retroviral particles carrying the IL-10 gene are produced in the supernatant of packaging cells transfected by these constructions. These particles can be used to infect mammalian cells, and thereby to induce the expression of IL-10 by these cells. Baculoviral vectors are used to produce larger quantities of recombinant protein, compared to retroviral vectors. Although this system of expression yields recombinant protein with glycosylation and disulphide-bridge, it induces altered posttranslational modifications and different glycosylations.

Another expression which can be used according to the invention is a fusion of nucleotidic sequences encoding human IL-10 and a strong promoter which will transfect eucaryotic cells, as described in the patent application WO94/00483.

As used herein, "effective amount of IL-10" means an amount sufficient at least to ameliorate or prevent a symptom of one of the following mentioned conditions. The effective amount for a particular patient may vary depending on such factors as the state of the condition being treated, the overall health of the patient, the method of administration, the severity of side-effects, and the like.

Analogs and/or agonists of IL-10 may be molecules which mimic IL-10 interaction with its receptors. Such may be analogs or fragmented IL-10, antibodies or portions thereof (such as Fab'₂ fragments), directed against ligands binding side epitopes of the IL-10 receptors, or anti-idiotypic antibodies (or portions thereof) against particular antibodies which bind to receptor-interacting portions of IL-10.

Antibodies or portions thereof can be raised to the IL-10 cytokine, fragments, and analogs, both in their naturally occurring forms and in their recombinant forms. Additionally, antibodies can be raised to IL-10 in either its active forms or in its inactive forms, the difference being that antibodies to the active cytokine are more likely to recognize epitopes which are only present in the active conformation. Anti-idiotypic antibodies or portions thereof are also contemplated in this method, and could be potential IL-10 agonists.

The medicament or the pharmaceutical composition according to the invention comprises also a pharmaceutical carrier. A pharmaceutical carrier can be any compatible non-toxic substance suitable for delivering the composition according to the invention to the patient. Generally, compositions useful for parenteral administration of such drugs are well known, and described in the patent application WO94/17773.

Preferably, "a disease involving the in vivo modulation of biological activity of interleukin-5" is a disease characterized by the presence of higher quantities of IL-5 in patients. The presence of higher quantities of IL-5 in patients means serum level of IL-5 above 100 pg/ml. For instance, asthmatic patients show serum level of IL-5 comprised between 200 and 1500 pg/ml, whereas normal individual values of 100 pg/ml or below are found (Korrigan et al., Annual Review Respirative Diseases, 147, pp. 540-547).

Advantageously, said disease is associated with eosinophilia, preferably selected among the group consisting of atopic diseases including allergic rhinitis and bronchial asthma, atopic dermatitis and other cutaneous diseases associated with eosinophil infiltration (including chronic eczema, erythema multiforme, dermatitis herpetiformis, mastocytosis, bullous disorders), Crohn's disease, bronchopulmonary aspergillosis, Spanish toxic oil syndrome, myalgiaeosinophilia syndrome induced by L-tryptophan preparations, certain drugs allergic reactions, tropical eosinophilia, helminthic diseases, Schulman syndrome (eosinophilic fasciitis), Churg-Strauss syndrome and other vasculitides with eosinophilia, Loeffler syndrome, chronic eosinophilic pneumonia, eosinophilic gastroenteritis, and the hypereosinophilic syndrome.

The Inventors have demonstrated in vivo that the i.p. injection of recombinant murin (rm) IL-10 (0.01 - 0.1 µg/cavity), concomitantly administered with ovalbumin, dose-dependently reduces allergic eosinophilia at 6, 24 and 48 hours after the challenge.

In addition, rmIL-10 is also effective in blocking antigen-induced IL-5 release in the peritoneal lavage fluid in vivo, as well as its in vitro generation by sensitized peritoneal cells cultured in the presence of ovalbumin. The inhibitory effect of rmIL-10 on antigen-induced eosinophilia and in vivo IL-5 release is suppressed when sensitized mice are pretreated with 1 mg/mouse of a neutralizing antibody against murin IL-10, indicating a specific biological effect for IL-10.

The Inventors show also that flow cytometry analysis revealed an increase of the proportion of CD4⁺ T-lymphocytes and in that of CD25⁺/CD4⁺ cells in the peritoneal lavage fluid collected 24 and 48 hours after challenge, respectively. No change in the number of CD8⁺ T-lymphocytes and in that of B cells is observed.

Thus, the Inventors have demonstrated unexpectedly that treatment of the sensitized mice with 0,1 µg rmIL-10 per mouse (20 g) reduced significantly CD4⁺ and CD25⁺/CD4⁺ T-lymphocytes accumulation in their peritoneal lavage fluid.

Thus, these findings indicate that rmIL-10 displays an anti-allergic activity in sensitized animals by preventing antigen-induced CD4⁺ T-lymphocytes and eosinophils accumulation, as well as IL-5 release in their peritoneal cavity.

Thus, the Inventors have for the first time discovered that the use of a pharmaceutical composition comprising an element chosen among the group consisting of interleukin-10 (IL-10), an analog and/or an agonist thereof for the manufacturing of a medicament may be used for the treatment and/or the prevention of diseases involving the in vivo modulation of the biological activity of interleukin-5, especially through the in vivo reduction of interleukin-5 release by T-lymphocytes (preferably the in vivo reduction of interleukin-5 serum level below 200 pg/ml), or through the in vivo reduction of antigen-induced recruitment (infiltration and accumulation) of eosinophils, and/or recruitment of CD4⁺ T-lymphocytes cells, particularly CD25⁺/CD4⁺ T-lymphocytes cells (which means more than 50% reduction of the number of these cells), in a patient.

In said medicament, the IL-10 is the sole active principle or is associated with interferon-α (IFN-α) and/or interferon-γ (IFN-γ).

A last aspect of the present invention concerns also a pharmaceutical composition comprising an effective amount of an element chosen among the group consisting of interleukin-10 (IL-10), an analog and/or an agonist thereof, an effective amount of an anti-histamine compound or an effective amount of a broncho-dilator chosen among the group consisting of β₂-agonists, phospho-diesterase inhibitors and/or a mixture thereof, and a pharmaceutically acceptable carrier for the prevention and/or the treatment of allergic diseases, preferably for the prevention and/or the treatment of bronchial asthma, allergic rhinitis and/or cutaneous erythema.

As used herein, "effective amount of IL-10 or of anti-histamine compound or of a broncho-dilator" means an amount sufficient at least to ameliorate or prevent a symptom of an allergic disease such as asthma. The effective amount for a particular patient may vary depending on such factors, as the state of the condition being treated, the overall health of the patient, the method of administration, the severity of side-effects, and the like.

Preferably, the effective amount of broncho-dilator used in the composition according to the invention is the standard amount used for the treatment of asthma or chronic asthma.

The composition according to the invention may be used, for instance, in a classic compressed-air-driven nebuliser.

Thus, the composition according to the invention may comprise between 10 et 1000 µg of IL-10, preferably around 400 µg, for a patient of 70 kg (one inhalation per day), and the amount of β₂-agonists is comprised between 10 and 2000 µg, preferably around 150 µg for a patient of 70 kg for one inhalation per day (see NIH Report No. 95-3659 (January 1995)).

### Short description of the drawings.

- The figure 1: represents kinetics of antigen-induced eosinophil infiltration in the peritoneal cavity of sensitized mice.
- The figure 2: shows the effect of rmIL-10 on antigen-induced eosinophil accumulation in the peritoneal lavage fluid from sensitized mice killed 6, 24 and 48 hours after challenge.
- The figure 3: shows the inhibition by rmIL-10 of antigen-induced IL-5 release in the peritoneal lavage fluid from sensitized mice.
- The figure 4: shows effect of anti-IL-10 monoclonal antibodies on the inhibition by rmIL-10 of antigen-induced IL-5 release in the peritoneal lavage fluid of sensitized mice. Mice were challenged i.p. either with saline or with 1 µg ovalbumin alone or concurrently administered with 0.1 µg rmIL-10. Antigen-challenged mice were treated i.v. with 1 mg/mouse of a specific anti-IL-10 monoclonal antibody described by Howard et al. (J. of Exp. Med., Vol. 177, pp. 1205-1208 (1993)), 1 hour prior to ovalbumin administration.
- The figure 5: shows the effect of rmIL-10 on the in vitro release of the IL-5 by peritoneal cells from sensitized mice cultured with 1 (panel a) or 10 or 100 µg/ml ovalbumin (panel b).

### Examples.

### Material and buffers

- sterile saline (Institut Pasteur, Paris, France);
- ovalbumin (5x crystallized, ICN Biomedicals, Inc., Costa Mesa, CA);
- heparin (Laboratories Choay, Paris, France);
- Diff-Quik stain (Baxter Dade AG, Dudingen, Switzerland);
- Al(OH)₃ (Merck, Darmstadt, Germany);
- rmIL-10 (Immugenex, Los Angeles, CA);
- heat-inactivated fetal calf serum (FCS, Boehringer Mannheim, Meylan, France);
- Trypan Blue (Rhone-Poulenc, Villers St-Paul, France);
- ³H thymidine (Amersham Life Science, Buckinghamshire, UK);
- 2-Mercaptoethanol, anti-CD4 FITC (clone H129.l9), anti-CD8a FITC (clone 53-6.7), anti-CD25 phycoerytrin (clone 3C7) and anti-CD45R FITC (clone RA3-6B2) (Sigma Chemical CO., St. Louis, MO);
- anti-CD3ε phycoerytrin (clone 145-2C11) (Pharmingen, San Diego, USA);
- rat IgG-FITC and rat IgG-phycoerytrin (Southern Biotechnology Associates, Birmingham, USA);
- streptomycin (Boehringer Mannheim, Germany);
- L-glutamine (Gibco, Life Technologies, Eragny, France);
- N-succinimidyl-4-(N-maleimido-methyl)-cyclohexane-l - carboxylate (SMCC);
- ammonium sulfate (Merck).

Buffers for cell culture were: Hanks' balanced solution without Ca²⁺ and Mg²⁺ (HBSS, pH 7.4) and RPMI 1640 (Gibco, Life Technologies, Eragny, France).

Buffer for flow cytometry was phosphate-buffered (0.01 M, pH 7.5, Merck) containing NaCl (0.15 M, Carlo Erba Reagenti, Milano, Italy) supplemented with 3% FCS and 0.1% of sodium azide (Merck).

Buffer for IL-5 determination by immunometric assay consisted of : 100 mM phosphate buffer, pH 7.4, containing 150 mM NaCl, 0.1% bovine serum albumin (BSA, fraction V, Sigma) and 0.01% sodium azide.

Acetylcholinesterase (AchE) was purified from the electric cell *Electrophorus electricus* by affinity chromatography (26). The characteristics of the preparation of the tetrametric form of this enzyme (G4 form) which was used for antibodies labelling, have been described elsewhere (1).

Rat IgG1 anti-mouse IL-10 antibody was described by Howard et al.

### Animals and sensitization procedure

Male Balb/C mice aged 8-weeks, weighing approximately 25 - 30 g (Janvier, Paris, France) were actively sensitized by a subcutaneous (s.c.) injection of 0.2 ml 0.9% w/v NaCl (saline) containing 100 µg ovalbumin adsorbed in 1.6 mg aluminium hydroxide. Seven days later, the animals received the same dose of ovalbumin in the presence of Al(OH)₃ and they were used one week thereafter.

### Antigen-induced peritonitis

Peritonitis was induced by the i.p. injection of 0.2 ml of a solution containing 5 µg/ml ovalbumin diluted in sterile saline (1 µg of ovalbumin, as final dose injected per cavity). Control animals received the same volume of sterile saline. At different time intervals after antigen challenge (1 - 48 h), animals were killed by an overdose of ether and the peritoneal cavity was opened and washed with 3 ml of heparinized saline (10 U/ml). Around 90% of the initial volume was recovered. In rare cases, when haemorrhages were noted in the peritoneal cavity, the animals were discarded.

This dose of ovalbumin and time-intervals after challenge have been selected on the basis of a previous study (2).

### Leucocyte analysis

Total leucocytes present in the peritoneal lavage fluids were counted in a Coulter counter ZM (Coultronics, Margency, France) and expressed as numbers of cells/ml. Differential cell counts were performed after cytocentrifugation (Hettich- Universal, Tuttlingen, Germany) and staining with Diff-Quik stain. At least 300 cells were counted and results were expressed as number of each cell population/ml.

### In vivo treatment

The effect of rmIL-10 on eosinophil infiltration into the peritoneal cavity was studied 6, 24 and 48 hours after antigen challenge. Sensitized mice were injected i.p. with 0.01 - 0.1 µg rmIL-10, diluted in 100 µl sterile saline and mixed with an equal volume of sterile saline containing 1 µg ovalbumin. In control experiments, sensitized mice were challenged either with saline or with 1 µg ovalbumin alone, as described above.

To study the effect of rmIL-10 on antigen-induced in vivo IL-5 release, mice were challenged with 1 µg ovalbumin in the presence or absence of 0.1 µg rmIL-10. 1, 3, 6 and 24 h after challenge, the animals were sacrificed and the peritoneal cavity was opened and washed, as described above. The peritoneal lavage fluid then centrifuged (300 x g, 10 min, 4 °C; Jouan, Saint Herblain, France) and cell free supernatant was stored at -20 °C until IL-5 determination.

In an attempt to verify the specificity of the effect of rmIL-10, mice were pretreated i.v. with 1 mg of the neutralizing antibody specific for murin IL-10, 2A5.l (3), or with its isotype matched control antibody, GL113 (4), 1 hour prior the i.p. injection of a saline solution containing 1 µg ovalbumin and 0.1 µg rmIL-10. Mice were sacrificed at 6 and 24 h for cell counting and IL-5 determination in the peritoneal lavage fluid.

### Cell culture and proliferation assay

Sensitized mice were sacrificed by an overdose of ether, the peritoneal cavity was opened and washed with 5 ml Hanks' balanced solution without Ca²⁺ and Mg²⁺ (pH 7.4) supplemented with 5% heat-inactivated FCS. Peritoneal cells were then washed twice (300 x g, 10 min, 4 °C) and resuspended in RPMI 1640 containing with 10% FCS, 2-mercaptoethanol (5 x 10⁻⁵ M), L-glutamine (2 mM) and streptomycin (200 µg/ml). Cell viability, which was determined by Trypan blue exclusion test, showed less than 2% of mortality. To determine the kinetics of antigen-induced IL-5 release, 2 x 10⁵ peritoneal cells/100 µl/well were cultured in 96-well microtiter plates (TPP, Europe), in the presence or absence of 100 µl of a solution containing 1-100 µg/ml ovalbumin diluted in RPMI. Cultures were maintained at 37 °C in a humidified atmosphere with 5% C0₂. After various time-intervals (6-120 h), plates were centrifuged (80 x g, 6 min, 4 °C), and supernatants were collected and stored at -20 °C until used.

The time of 48 h after antigen stimulation was selected to study the effect of rmIL-10 on IL-5 release. To do so, peritoneal cells stimulated with 1 - 100 µg/ml ovalbumin were cultured in the presence or in the absence of 10 - 100 ng/ml rmIL-10. In separate experiments, 30 h after the beginning of culture, 0.5 µCi/well ³H thymidine were added and cultures were pulsed for 18 h. Peritoneal cell proliferation was evaluated by measuring incorporated radioactivity using a Betaplate Berthold β counter (Bad Wildbad, Germany).

### Measurement of cytokine production by enzyme immunometric assay

The levels of IL-5 in the culture supernatants or peritoneal lavage fluid from sensitized saline- or ovalbumin-challenged untreated or 0.1 µg rmIL-10-treated mice were determined by enzyme immunometric assay. This method involves the reaction of thiol groups of TRFK-5 Fab' fragments with maleimido groups previously introduced into acetylcholinesterase (5). Maleimido groups were first incorporated into acetylcholinesterase by reaction with the heterobifunctional reagent N-succinimidyl-4-(N-maleimidomethyl)-cyclohexane-l-carboxylate (SMCC).

TRFK-S and TRFK-4 antibodies were purified from ascitic fluids described by Schumacher et al. (J. Immunol., Vol. 14, p. 1571 (1988)), using affinity chromatography method on a protein G column (HiTrap affinity columns, Pharmacia Biotechnology, Uppsala, Sweden) after precipitation by ammonium sulfate. Characteristics of these antibodies were described in detail elsewhere (6). The purity of this preparations was assessed by polyacrylamide gel electrophoresis techniques under denaturating and non-reduction conditions. F(ab')2 fragments were obtained from purified antibodies by treatment with pepsin in acidic medium (7). Fab' fragments were further obtained from F(ab')2 by reduction in presence of 2-mercaptoethanolamine. The thiol content of the Fab' preparation was determined colorimetrically after reaction with 3,5'-dithiobisnotrobenzoic acid (DTNB), as described by Ellmart et al. (8).

Immunometric assays were performed in 96-well microtiter plates (MaxiSorps, Nunc, Roskilde Danmarck), coated with 10 µg/ml of the rat monoclonal antibody for murine IL-5 (TRFK-4) as described previously (9). The one-step procedure used for immunometric assays involved the simultaneous addition of 100 µl of IL-5 standards (7.8-1,000 pg/ml) or samples, with 100 µl of the second rat anti-IL-5 antibody, TRFK-5-acetylcholinesterase conjugated, at the concentration of 10 Ellman units/ml. After incubation for 18 h at 4 °C, the plates were then extensively washed and solid-phase bound acetylcholinesterase activity was determined colorimetrically by addition of 200 µl of Ellman's reagent (10). Absorbency was read at 405 nm with an automatic microplate reader (Dinatech MR 5000; Dinatech Laboratories, Saint Cloud, France). The lower limit of detection of this assay is approximately of 5 pg IL-5/ml sample.

### Immunofluorescence analysis

The immunophenotyping of murine peritoneal cells from saline- or ovalbumin-challenged untreated or rmIL-10 (0.1 µg)-treated mice was analyzed by flow cytometry. Briefly, peritoneal lavage cells collected 6, 24 and 48 h after antigen challenge were washed twice in PBS containing 3% FCS and 0.1% of sodium azide. Cells (3 x 10⁵) were subsequently stained. The monoclonal antibodies used were as follows: rat IgG anti-CD4 FITC, rat IgG anti-CD8a FITC, hamster IgG anti-CD3ε phycoerytrin (PE), rat IgG anti-CD25 phycoerytrin, rat IgG anti-CD45R FITC, or the corresponding isotype matched controls. Stained cells were resuspended in phosphate-buffered saline containing 3% FCS and 0.1% of sodium azide. Cell samples were analyzed on a FACScan flow cytometer (Becton-Dickinson Immunocytometry System, CA). For each sample, 10,000 events were collected at 1000 sec and three-colour listmode data analysis was performed using Lysis II software (Becton-Dickinson). Peritoneal lavage fluids were further analyzed on the basis of light scatter properties in which the relative size (forward light scatter) and granularity (side angle scatter) of individual cell population was defined.

### Statistical analysis

Data were analyzed statistically using a microcomputer programme and an analysis of variance (ANOVA), followed by Student's t-test for unpaired values. *p* values of 0.05 or less were considered significant. The results are expressed as means ± SEM of the indicated number of experiments.

### Antigen-induced cell accumulation into the peritoneal cavity of sensitized mice

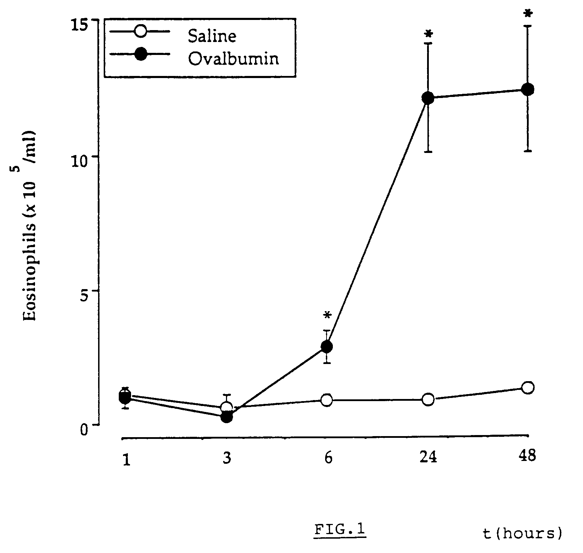

The i.p. injection of 1 µg ovalbumin to sensitized Balb/C mice induced a marked increase in the number of eosinophils, starting at 6 h and reaching a plateau between 24 and 48 h (Fig. 1). One week later, eosinophils counts returned to basal levels. No changes in the number of eosinophils were observed in saline-challenged mice at any time point considered (Fig. 1).

### Example 1 : Effect of rmIL-10 on antigen-induced eosinophil recruitment into the peritoneal cavity of sensitized mice

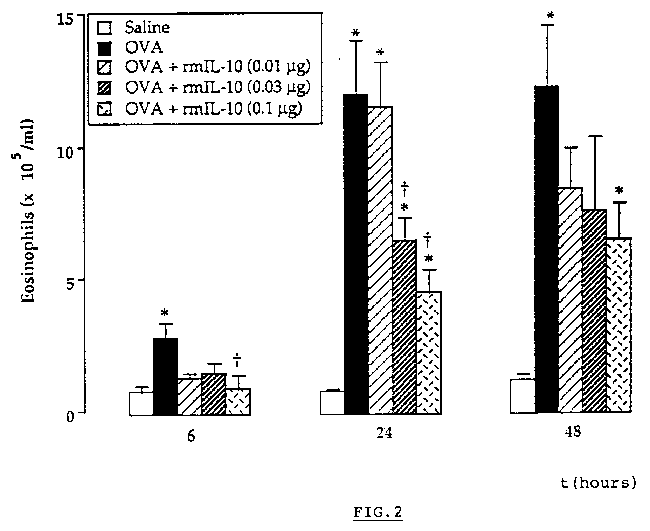

The administration of rmIL-10 (0.01 - 0.1 µg), concomitantly injected with 1 µg ovalbumin, reduced eosinophil counts at 6 h. This effect was not dose-dependent and significant inhibition was achieved with the dose 0.1 µg rmIL-10 (Fig. 2). Allergic eosinophilia at 24 h was significantly and dose-dependently inhibited by the treatment of sensitized mice with 0.03 and 0.1, but not 0.01 µg rmIL-10. Indeed, reductions of 45% and 62% in the number of eosinophils were observed in animals treated with 0.03 and 0.1 µg rmIL-10, respectively (Fig. 2). Even though a 47% inhibition of allergic eosinophilia at 48 h was noted upon treatment with 0.1 µg rmIL-10, the high variability in cell counts precluded the results from achieving statistical significance (Fig. 2).

### Example 2 : Effect of rmIL-10 on antigen-induced changes in T and B cell populations in the peritoneal cavity of sensitized mice

To determine the presence and the phenotype of lymphocytes in the peritoneal cavity of antigen-challenged mice, peritoneal cells were stained and analyzed by flow cytometry. Antigen challenge was followed by a rise in the percent of CD4⁺ T-cells at 24 and 48 h (Table 1). A time-dependent increase in the proportion of CD4⁺ cells bearing IL-2 receptor on their surface (CD25⁺/CD4⁺ T-cells) was noted, the maximum being reached 48 h after antigen challenge. Conversely, neither changes in the percent of CD8⁺ T-lymphocytes nor in that of B220⁺ B cells were observed when peritoneal cells from saline- and ovalbumin-challenged mice were compared at all time points (Table 1).

**Table 1**

| Effect of the i.v. administration of 1 mg/mouse of the anti-IL-10 monoclonal antibody (mAb) described by Howard et al. on antigen-induced eosinophil accumulation into the peritoneal fluid from sensitized rmIL-10 (0.1 µg)-treated mice. | | | | |
|---|---|---|---|---|
| **Challenge intraperitoneal** | **Treatment intraperitoneal** | **Treatment intravenous** | **Eosinophils x 10**^{**4**} **ml of peritoneal fluid** | |
| | | | **6 h** | **24 h** |
| Saline | None | None | 1.0 ± 0.1 | 1.1 ± 0.2 |
| Ovalbumin | None | None | 1.9 ± 0.2* | 7.9 ± 1.9* |
| Ovalbumin | rmIL-10 | mAb | 0.5 ± 0.2** | 1.6 ± 0.3** |
| Ovalbumin | rmIL-10 | 2A5.1 | 2.0 ± 0.3*° | 12.1 ± 1.8*° |
| Values are means ± SEM of 6-12 experiments | | | | |

| | | | | |
|---|---|---|---|---|
| * P<0.05, as compared to saline-challenged untreated mice | | | | |
| ** P<0.05, as compared to ovalbumin-challenged untreated mice | | | | |
| ° P<0.05, as compared to ovalbumin-challenged GL113-treated mice | | | | |

When 0.1 µg rmIL-10 were co-injected with ovalbumin, a significant reduction in the percent of CD4⁺ T-cells at 24 h and in that of CD25⁺/CD4⁺ cells at 48 h was observed (70% and 43% of inhibition, respectively, n= 4, P<0.01) (Table 1).

### Example 3 : Effect of rmIL-10 on antigen-induced IL-5 release

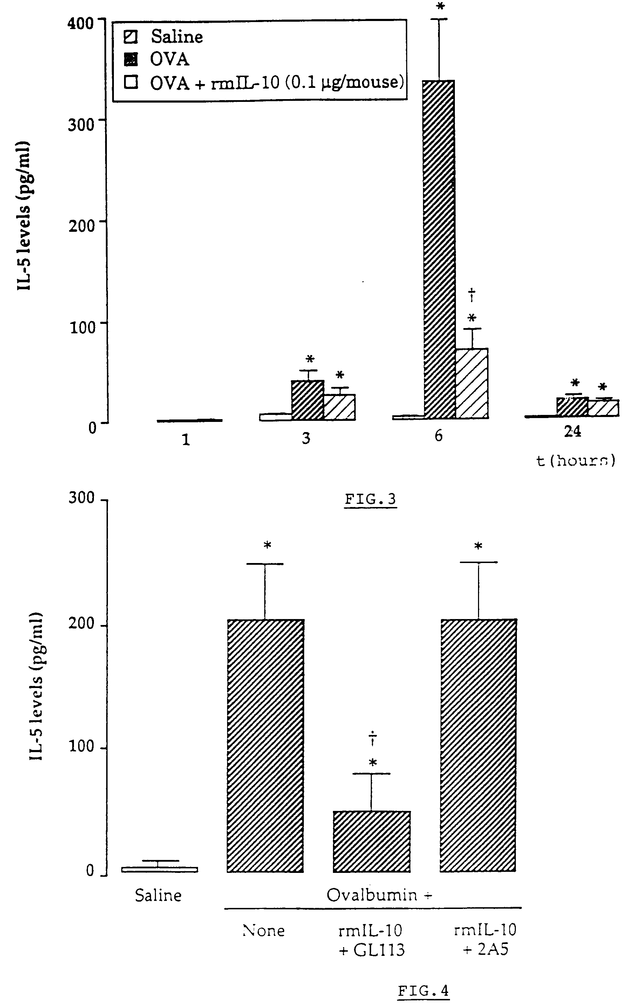

The i.p. injection of 1 µg ovalbumin to sensitized mice induced a marked release of IL-5 in the peritoneal lavage fluid, which started at 3 h, peaked at 6 h and decreased thereafter (Fig. 3). The levels of IL-5 in the peritoneal lavage fluid from saline-injected mice killed at all time points never exceeded 5.6 ± 2.1 pg/ml (n= 8-14, Fig. 3).

The administration of 0.1 µg rmIL-10 along with ovalbumin, drastically reduced IL-5 release in the peritoneal lavage fluid at 6 h (79% inhibition, n= 9; P<0.05), without affecting that observed at 3 h (Fig. 3). The ELISAs determination for TNF-α, granulocyte-macrophage colony stimulating factor (GM-CSF) and IFN-γ failed to show increased levels of these cytokines in the peritoneal lavage fluid of ovalbumin as opposed to saline-challenged mice.

### Example 4 : Effect of anti-IL-10 mAb on the rmIL-10-mediated inhibition of a of antigen-induced eosinophil infiltration and IL-5 release in the peritoneal lavage fluid

The i.v. injection of the rat anti-mouse IL-10 monoclonal antibody described by Howard et al., but not of an isotype matched control, GL113, 1 h prior to the administration of 0.1 µg rmIL-10 and 1 µg ovalbumin, suppressed the inhibitory activity evoked by rmIL-10 on eosinophil accumulation in the peritoneal lavage fluid at 6 and 24 h. In parallel, the monoclonal antibody anti-IL-10 also abolished the blockade by rmIL-10 of allergic IL-5 release at 6 h, indicating that the effect of IL-10 on eosinophil infiltration and IL-5 release in the peritoneal lavage fluid are due exclusively to its biological activity (Fig. 4).

### Example 5 : Effect of rmIL-10 on antigen-induced IL-5 release by mouse peritoneal cells

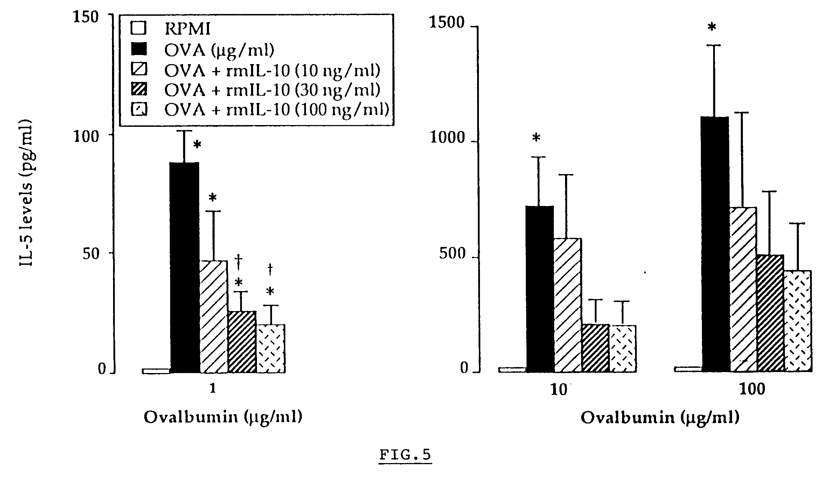

Incubation of peritoneal cells from sensitized mice with 1 µg/ml ovalbumin in the presence of 10 - 100 ng/ml rmIL-10 was followed by a significant reduction in the levels of IL-5 at 48 h. Indeed, 70% and 77% inhibitions were observed for the concentrations of 30 and 100 ng/ml rmIL-10, respectively (n=5, P<0.05; Fig. 5a). In contrast, rmIL-10 failed to decrease significantly IL-5 generation induced by higher concentrations of ovalbumin (10 - 100 µg/ml) even though 72% and 39% inhibitions were detected with 100 ng/ml rmIL-10, respectively (Fig. 5b).

Finally, the inventors tested whether the inhibitory effect of IL-10 on IL-5 release would be related to a blockade of antigen-induced T-cell proliferation. Peritoneal cell culture during 48 h in the presence of 10 or 100 µg/ml ovalbumin failed to elicit any proliferative response. Indeed, RPMI-treated peritoneal cells showed 129 ± 27 cpm of ³H thymidine incorporation (n=6), while amounts of 137 ± 26 and 184 ± 36 cpm were detected in ovalbumin (10 and 100 µg/ml)-stimulated cells, respectively (n=6). Under these conditions, incubation with 100 ng/ml rmIL-10 did not modify ³H thymidine incorporation by peritoneal cells challenged with 10 or 100 µg/ml ovalbumin.

### REFERENCES.

1. **Grassi, J., Frobert, Y., Lamourette, P. and Lagoutte, B.** (1988) *Anal. Bioch.* 168:436.
2. **Zuany-Amorim, C., Leduc, D., Vargaftig, B.B. and Pretolani, M.** (1993) *Br. J. Pharmacol.* 110:917.
3. **Howard, M., Muchamuel, T., Andrade, S. and Menon, S.** (1993) *J. Exp. Med.* 177:1205.
4. **Grassi, J., Frobert, Y., Pradelles, P., Chercuitte, D., Gruaz, D., Dayer, J.M. and Poubelle, P.** (1989) *J. Immunol. Methods* 123:193.
5. **Schumacher, J.H., O'Garra, A., Shrader, B., van Kimmenade, A., Bond, M.W., Mosmann, T.R. and Coffman, R.L.** (1988) *J. Immunol.* 141:1576.
6. **Lamoyi, E. and Nisonoff, A.** (1983) *J. Immunol. Methods* 56:235.
7. **Ellman, G.** (1959) *Arch. Biochem. Biophys.* 82:7.
8. **Pradelles, P., Grassi, J. and Maclouf, J.** (1985) *Anal. Chem.* 57:1170.
9. **Ellman, G., Courtney, K., Andres, V. and Featherstone, R.** (1961) *Biochem. Pharmacol.* 7:88.
10. **Garlisi, C.G., Falcone, A., Kung, T.T., Stelts, D., Pennline, K.J., Beavis, A.J., Smith, S.R., Egan, R.W. and Umland, S.P.** *Clin. Immunol. Immunopathol.* 75:75.

## Claims

1. Use of an element chosen among the group consisting of interleukin-10, an analog and/or an agonist thereof, for the manufacturing of a medicament, for the treatment and/or the prevention of a disease involving the in vivo modulation of the biological activity of interleukin-5.

2. Use according to claim 1, for the manufacturing of a medicament, for the treatment and/or the prevention of a disease involving the in vivo presence of high quantities of interleukin-5 in a patient, such as a disease associated with eosinophilia.

3. Use according to claim 2, for the manufacturing of a medicament for the treatment and/or the prevention of a disease selected from the group consisting of atopic diseases including allergic rhinitis and bronchial asthma, atopic dermatitis and other cutaneous diseases associated with eosinophil infiltration (including chronic eczema, erythema multiforme, dermatitis herpetiformis, mastocytosis, bullous disorders), Crohn's disease, broncho-pulmonary aspergillosis, Spanish toxic oil syndrome, myalgiaeosinophilia syndrome induced by L-tryptophan preparations, certain drugs allergic reactions, tropical eosinophilia, helminthic diseases, Schulman syndrome (eosinophilic fasciitis), Churg-Strauss syndrome and other vasculitides with eosinophilia, Loeffler syndrome, chronic eosinophilic pneumonia, eosinophilic gastroenteritis, and the hypereosinophilic syndrome.

4. Use according to any of the preceding claims, for the manufacturing of a medicament for the in vivo reduction of interleukin-5 serum level, preferably below 200 pg/ml, in a patient.

5. Use according to any of the preceding claims, for the manufacturing of a medicament for the in vivo reduction of recruitment of CD4⁺ T-lymphocytes, especially CD25⁺/CD4⁺ T-lymphocytes in a patient.

6. Use according to any of the preceding claims, for the manufacturing of a medicament for the in vivo reduction of the recruitment, infiltration and accumulation of eosinophils in a patient.

7. Use according to any of the preceding claims, wherein the IL-10 is the sole active principle in a medicament.

8. Use according to any of the preceding claims, wherein IL-10 is associated in the medicament with interferon-α (IFN-α) and/or with interferon-γ (IFN-γ).

9. Use according to any of the preceding claims, wherein the IL-10 is recombinant human IL-10.

10. Use according to any of the preceding claims, wherein the IL-10 is viral IL-10, an IL-10 peptide or a chimeric protein derived from both cellular and viral IL-10.

11. Pharmaceutical composition comprising an effective amount of an element chosen among the group consisting of interleukin-10, an analog and/or an agonist thereof, an effective amount of a broncho-dilator chosen among the group consisting of β₂-agonists, phospho-diesterase inhibitors and/or a mixture thereof and a pharmaceutically acceptable carrier, for the treatment and/or the prevention of allergic diseases.

12. Pharmaceutical composition according to claim 11, for the treatment and/or the prevention of bronchial asthma.

13. Pharmaceutical composition comprising an effective amount of an element chosen among the group consisting of interleukin-10, an analog and/or an agonist thereof, an effective amount of an antihistamine compound and a pharmaceutically acceptable carrier for the treatment and/or the prevention of allergic diseases.

14. Pharmaceutical composition according to claim 13, for the treatment and/or the prevention of allergic rhinitis and/or cutaneous erythema.
